# EUROPEAN PATENT APPLICATION

(11) **EP 3 360 541 A1**
(43) Date of publication of application: **15.08.2018**
(21) Application number: 18156100.2
(22) Date of filing: 09.02.2018
(51) Int. Cl.: A61K 9/20, A61K 9/24, A61K 31/397, A61K 31/40, A61K 31/405

(54) **PHARMACEUTICAL BILAYER TABLET COMPOSITION OF ATORVASTATIN CALCIUM AND EZETIMIBE**

(30) Priority: 13.02.2017 TR 201702101
(71) Applicant: Sanovel Ilac Sanayi ve Ticaret A.S., 34460 Istanbul (TR)
(72) Inventor: TÜRKYILMAZ, Ali, 34460 Istanbul (TR); YILDIRIM, Ediz, 34460 Istanbul (TR); TOK, Gülcin, 34460 Istanbul (TR); BAS, Oguz, 34460 Istanbul (TR); KÖKSAL UZUN, Selin, 34460 Istanbul (TR)
(74) Representative: Sevinç, Erkan

(57) **Abstract**

The present invention relates to a pharmaceutical bilayer tablet composition comprising atorvastatin calcium combined with ezetimibe with at least one polymer.

## Description

### Field of the invention

The present invention relates to a pharmaceutical bilayer tablet composition comprising atorvastatin calcium combined with ezetimibe with at least one polymer.

### Background of invention

The chemical name of atorvastatin is (3R,5R)-7-[2-(4-fluorophenyl)-3-phenyl-4-(phenylcarbamoyl)-5-propan-2-ylpyrrol-1-yl]-3,5-dihydroxyheptanoic acid and has a chemical structure which is shown in the Formula I.

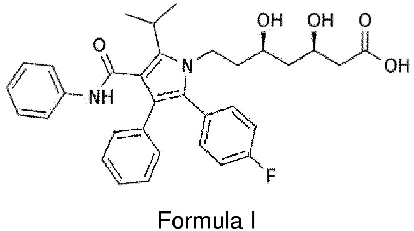

Atorvastatin belongs to the statin lipid regulating drugs, it is a HMG-CoA reductase inhibitor. Atorvastatin is reductase competitively in the body, reducing the synthesis of cholesterol, but also LDL receptor synthesis the main site of action in the liver, resulting in blood cholesterol and low-density lipoprotein cholesterol levels, a moderate decrease in serum triglyceride levels, and elevated blood levels of HDL.

Ezetimibe is in a class of compounds known as lipid-lowering compounds that selectively inhibits the intestinal absorption of cholesterol and related phytosterols. Its chemical name is (3R,4S)-1-(4-fluorophenyl)-3-[(3S)-3-(4-fluorophenyl)-3-hydroxypropyl]- 4-(4-hydroxy-phenyl) azetidin-2-one and has a chemical structure which is shown in the Formula II.

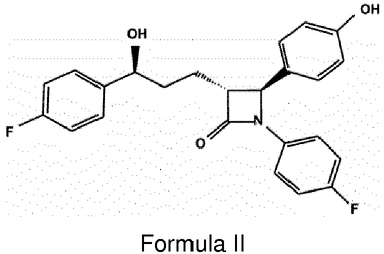

Ezetimibe reduces blood cholesterol by inhibiting the absorption of cholesterol by the small intestine. Its mechanism of action differs from those of other classes of cholesterol-reducing compounds, such as HMG-CoA reductase inhibitors. It doesn't inhibit cholesterol synthesis in the liver, or increase bile acid excretion but inhibits the absorption of cholesterol, leading to a decrease in the delivery of intestinal cholesterol to the liver. Such mechanism is complementary to that of HMG-CoA reductase inhibitors.

When atorvastatin combined with ezetimibe, it is effective than atorvastatin alone, so that the plasma levels of LDL-C was significantly reduced, the difference was statistically significant, and atorvastatin and ezetimibe combined with the use of a double dose atorvastatin compared the efficacy of lowering LDL-C similar difference was statistically significant. Thus, atorvastatin combined with ezetimibe and atorvastatin alone compared, can reduce LDL-C to obtain additional benefits, can make more high-risk or very high risk of CHD patients with blood lipid standards. Statins likely to cause liver function, myopathy and GI and the like.

In prior art, there are other pharmaceutical dosage forms comprising atorvastatin calcium and ezetimibe, but it is needed to develop a formulation which has a desired disintegration time and long-term stability for use and also the process described in prior art are complex, time-consuming and costs are high.

Atorvastatin calcium is an unstable substance sensitive to heat, humidity, low pH of the environment, and light, particularly UV radiation. Carbon dioxide is thought to be the most important factor leading to the instability of the drug. Its effect is ascribed to the lowering of pH, which results in the decomposition of hydroxyl acids, particularly to their lactones.

The stability of atorvastatin calcium can be improved, simply, by elevating the pH, but, it was pointed out that gastric troubles may be caused if a medicine with a high content of alkaline substance is administered to patients.

At this present invention, to improve the stability of the atorvastatin calcium, calcium carbonate is used as stabilizers. It relates to reducing the amount of water in the formulation, which is both inconvenient and also difficult to achieve for long term stability.

In the prior art, ezetimibe has low solubility. This also affects this bioavailability negatively.

At this present invention, high amount of filler and at least one polymer is used in the second layer of bilayer tablet which comprises ezetimibe, so this overcomes the problems of stability problems and solubility problems of ezetimibe.

Finally, at this present invention, formulation overcomes the above significant development challenges and enables a fixed combination formulation that has simultaneously:
a) a better dissolution profile to individual active ingredients,
b) a stable formulation despite the incompatibilities of two component molecules - atorvastatin calcium and ezetimibe
c) eliminates especially the low solubility problem of ezetimibe and the stability problem of atorvastatin calcium to enhance the good dissolution profile and good shell-life of the final pharmaceutical product.

### Detailed description of the invention

The main objectof the present invention is to provide atorvastatin calcium and ezetimibe bilayer tablet to provide high stability and solubility.

Another object of the present invention is to provide desired level of dissolution rate and compressibility which overcomes the above described problems in prior art and have additive advantages over them.

A further object of the present invention is to stabilize the active pharmaceutical ingredients which does not degrade to unwanted impurities.

Another object of the present invention is to provide pharmaceutical formulations comprising active compounds finely and homogenously dispersed in one or more polymeric carriers that are produced by wet granulation process.

According to one embodiment of the present invention, the pharmaceutical bilayer tablet comprises;
a. first layer comprising atorvastatin calcium
b. second layer comprising ezetimibe
wherein the weight ratio of the second layer to the first layer is between 0.05 and 8.0, further comprising at least one polymer.

According to one embodiment of the present invention, the weight ratio of the second layer to the first layer is between 0.1 and 5.0.

According to one embodiment of the present invention, the amount of atorvastatin calcium in the tablet is 0.5% to 10.0% by weight and the amount of ezetimibe in the tablet is 0.5% to 10.0% by weight.

Suitable polymers are selected from the group comprising hydroxypropylcellulose, hydroxypropylmethylcellulose, polysorbate (i.e polysorbate 80), polyvinylpyrrolidone (PVP), vinyl acetate, cellulose acetate phthalate, polyethylene oxide, polypropylene oxide, copolymers of ethylene oxide, propylene oxide, methacrylic acid/ethyl acrylate copolymers, methacrylic acid/methyl methacrylate copolymers, butyl methacrylate/2-dimethylaminoethyl methacrylate copolymers, polyacrylamides, vinyl acetate polymers, polyvinyl alcohol, hydroxypropylmethylcellulose succinate, hydroxypropylcellulose acetate phthalate, hydroxypropylcellulose acetate succinate, hydroxypropylmethylcellulose acetate phthalate, hydroxypropylmethylcellulose acetate succinate, hydroxypropylmethylcellulose acetate trimellitate, hydroxypropylcellulose butyrate phthalate, methylcellulose acetate phthalate, cellulose acetate trimellitate, cellulose acetate terephthalate or mixtures thereof, preferably the polymers are hydroxypropylcellulose, hydroxypropylmethylcellulose and polysorbate.

According to this embodiment of the present invention, the amount of polymer(s) in the tablet is 0.5% to 5.0% by weight.

According to this embodiment of the present invention, each layer comprises at least one polymer.

According to this embodiment of the present invention, the weight ratio of polymer(s) in the second layer to polymer(s) in the first layer is 0.1-10.0, preferably the ratio is 0.2-6.0.

According to this embodiment of the present invention, polysorbate, especially polysorbate 80 is very effective excipient in the preparation of the tablet. It has been surprisingly found that the object of the present invention is achieved when polysorbate 80 and hydroxypropylcellulose are used in a certain ratio in a layer. Consequently, the ratio of hydroxypropylcellulose to polysorbate 80 is 0.5-10.0 by weight, preferably the ratio is 2.1-8.0. Said ratio provides adequate disintegrating properties for the tablets of the present invention but also stabilizes the active pharmaceutical ingredient which does not degrade to unwanted impurities.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active ingredients and the excipients.

The pharmaceutical bilayer tablet further comprises pharmaceutically acceptable excipients which are selected from the group comprising fillers, disintegrants, surface active agents, lubricants, stabilizers or mixtures thereof.

Suitable fillers are selected from the group comprising microcrystalline cellulose, lactose monohydrate, starch, sucrose, glucose, dextrose, maltodexrin, natural and synthetic gums (e.g. acacia tree), gelatin, pregelatinized starch, polyvinylpyrrolidone, cellulose derivatives or mixtures thereof, preferably fillers are microcrystalline cellulose and lactose monohydrate. They maintain the stability of the active ingredients shelf-life.

According to this embodiment of the present invention, the amount of the fillers in tablet is 12.0%-65.0% by weight.

According to this embodiment of the present invention, wherein the weight ratio of microcrystalline cellulose in the second layer to microcrystalline cellulose in the first layer is 0.05-10.0, preferably the ratio is 0.1-5.0.

Suitable disintegrants are selected from the group comprising croscarmellose sodium, sodium starch glycolate, alginic acid, crospovidone, sodium alginate, starch (e.g., corn, potato) or mixtures thereof, preferably disintegrant is croscarmellose sodium.

Suitable surface active agents are selected from the group comprising sodium lauryl sulphate, polyoxyethylene glycol esters, sulphate or mixtures thereof, Preferably the agent is sodium lauryl sulphate which avoids crystallization of ezetimibe, and thus the dissolution of ezetimibe is not affected adversely.

Suitable lubricants are selected from the group comprising silica colloidal anhydrous, sodium stearyl fumarate, magnesium stearate, talc, polyethylene glycol, stearic acid, aluminum silicate or mixtures thereof, preferably lubricants of this invention are silica colloidal anhydrous, sodium stearyl fumarate, magnesium stearate.

Suitable stabilizers are selected from the group comprising calcium carbonate, calcium hydroxide, dibasic calcium phosphate, tribasic calcium phosphate, calcium sulphate, calcium acetate, calcium gluconate, calcium glycerol phosphate, magnesium carbonate, magnesium hydroxide, magnesium sulphate, magnesium acetate, magnesium silicate, magnesium aluminate or mixtures thereof. Preferably the stabilizer is calcium carbonate whose total amount in the tablet is 1.0%- 10.0%, preferably 2.0%-8.0% by weight.

Finally, each individual layer compressed into a tablet. the two components are more stable. Furthermore, the tablet is coated with film coating.

The pharmaceutical bilayer tablet composition comprises film coating to protect the composition against the moisture and light to maintain the stability. Suitable coating ingredients are selected from the group comprising hydroxypropylmethylcellulose, polyethylene glycol (PEG), talc, titanium dioxide, polyvinyl alcohol (PVA), polyvinyl alcohol-polyethylene glycol copolymers, polyvinylprolidone, polyvinylprolidone-vinyl acetate copolymer (PVP-VA), pigments, dyes, iron oxide or mixtures thereof, preferably hydroxypropylmethylcellulose, polyethylene glycol, talc, titanium dioxide. The amount of the film coating is 0.5%-4% by weight.

According to the present invention, the pharmaceutical bilayer tablet formulation comprises the following ingredients;

Atorvastatin layer: atorvastatin calcium at 1.0-40.0% by weight, microcrystalline cellulose at 20.0-50.0% by weight, calcium carbonate at 12.0- 38.0% by weight, lactose monohydrate at 12.0-35.0% by weight, croscarmellose sodium at 1.0-17.0% by weight, hydroxypropylcellulose at 0.5-11.0% by weight, polysorbate at 0.01- 5.0 by weight, magnesium stearate at 0.01-8.0% by weight, silica colloidal anhydrous at 0.01-8.0% by weight, pure water0.001 -10.0% by weight.

Ezetimibe layer: ezetimibe at 1.0-20.0% by weight, microcrystalline cellulose at 20.0-60.0% by weight, lactose monohydrate at 20.0-42.0% by weight, croscarmellose sodium at 3.0-20.0% by weight, hydroxypropylmethylcellulose at 0.1-12.0% by weight, sodium lauryl sulfate at 0.1-10.0% by weight, sodium stearyl fumarate at 0.1-10.0% by weight, silica colloidal anhydrous at 0.01-8.0% by weight, pure water 0.001-10.0% by weight.

Another embodiment of the present invention is the manufacturing process of the pharmaceutical tablet compositions of atorvastatin calcium and ezetimibe. The pharmaceutical tablet compositions of atorvastatin calcium and ezetimibe of the present invention can be prepared in a fast, efficient and commercially cost low manufacturing process.

The tablets may be prepared by a granulation process. A characteristic of the methods given below based on wet granulation and used for the production of the formulations of the present invention. Suitable granulation solutions are selected from a group comprising pure water, ethyl alcohol, glycerin, sorbitol, polyethylene glycol, propylene glycol, isopropyl alcohol, or mixtures thereof, preferably granulation solution is pure water.

Another embodiment of the present invention is to provide two different processes for preparing the pharmaceutical bilayer tablet composition for ezetimibe layer comprising the following steps:

### 1^{st} one is:

### Ezetimibe layer:

a) mixing lactose monohydrate and half of croscarmellose sodium,
b) adding hydroxypropylmethylcellulose, sodium lauryl sulfate and ezetimibe in aqueous solution to step (a)
c) drying the granules
d) granulating the powder mixture with pure water
e) drying the granules
f) sieving the dried granules through a sieving mesh
g) adding microcrystalline cellulose, the remaining part of croscarmellose sodium, silica colloidal anhydrous to step (f) mixture and mixing them
h) adding sodium stearyl fumarate to step (g) mixture and mixing them.

### 2^{nd} one is:

### Ezetimibe layer:

a) mixing ezetimibe, sodium lauryl sulfate, hydroxypropylmethylcellulose (E5 LV), lactose monohydrate, half of croscarmellose sodium,
b) granulating the powder mixture with pure water
c) drying the granules
d) sieving the dried granules through a sieving mesh
e) adding microcrystalline cellulose, the remaining part of croscarmellose sodium, silica colloidal anhydrous to step (d) mixture and mixing them
f) adding sodium stearyl fumarate to step (e) mixture and mixing them.

Another embodiment of the present invention is to provide a process for preparing the pharmaceutical bilayer tablet composition for atorvastatin calcium layer comprising the following steps:

### Atorvastatin layer:

a) mixing atorvastatin calcium, microcrystalline cellulose, calcium carbonate, lactose monohydrate, half of croscarmellose sodium,
b) adding hydroxypropylcellulose, polysorbate in aqueous solution to step (a) mixture
c) sieving the wet granulating
d) drying the granules and sieving the mixture
e) adding the remaining part of croscarmellose sodium, silica colloidal anhydrous to step (d) mixture and mixing them
f) adding magnesium stearate to step (e) mixture and mixing them

### Example 1: Bilayer tablet

| **First Layer** | **(%) amount (w/w)** |
|---|---|
| Atorvastatin calcium | 5.0- 20.0 |
| Microcrystalline cellulose Ph101 | 28.0- 43.0 |
| Calcium carbonate | 15.0-30.0 |
| Lactose monohydrate | 15.0-30.0 |
| Croscarmellose sodium | 3.0-13.0 |
| Hydroxypropylcellulose (LF) | 1.0-7.0 |
| Polysorbate 80 | 0.1-3.0 |
| Magnesium stearate | 0.1-5.0 |
| Silica colloidal anhydrous | 0.1-5.0 |
| **Total (First layer)** | **100** |

| **Second Layer** | **(%) amount (w/w)** |
|---|---|
| Ezetimibe | 3.0-12.0 |
| Microcrystalline cellulose | 37.0-50.0 |
| Lactose monohydrate | 25.0-35.0 |
| Croscarmellose sodium | 5.0-15.0 |
| Hydroxypropylmethylcellulose E5 LV | 1.0-9.0 |
| Sodium lauryl sulfate | 1.0-6.0 |
| Sodium stearyl fumarate | 0.8-4.0 |
| Silica colloidal anhydrous | 0.05-5.0 |
| **Total (Second Layer)** | **100** |

### Process for example 1

### Ezetimibe layer:

a) mixing lactose monohydrate and half of croscarmellose sodium,
b) adding hydroxypropylmethylcellulose, sodium lauryl sulfate and ezetimibe in aqueous solution to step (a)
c) drying the granules
d) granulating the powder mixture with pure water
e) drying the granules
f) sieving the dried granules through a sieving mesh
g) adding microcrystalline cellulose, the remaining part of croscarmellose sodium, silica colloidal anhydrous to step (f) mixture and mixing them
h) adding sodium stearyl fumarate to step (g) mixture and mixing them.

### Atorvastatin layer:

a) mixing atorvastatin calcium, microcrystalline cellulose, calcium carbonate, lactose monohydrate, half of croscarmellose sodium,
b) adding hydroxypropylcellulose, polysorbate in aqueous solution to step (a) mixture
c) Sieving the wet granulating
d) drying the granules and sieving the mixture
e) adding the remaining part of croscarmellose sodium, silica colloidal anhydrous to step (d) mixture and mixing them
f) adding magnesium stearate to step (e) mixture and mixing them
   - After, pressing these two mixtures for each layer into bilayer tablets,
   - Coating these tablets.

### Example 2: Bilayer tablet

| **First Layer** | **(%) amount (w/w)** |
|---|---|
| Atorvastatin calcium | 8.0- 15.0 |
| Microcrystalline cellulose Ph101 | 32.0- 41.0 |
| Calcium carbonate | 18.0-28.0 |
| Lactose monohydrate | 18.0-25.0 |
| Croscarmellose sodium | 4.0-10.0 |
| Hydroxypropylcellulose (LF) | 1.0-5.0 |
| Polysorbate 80 | 0.2-2.0 |
| Magnesium stearate | 0.2-3.0 |
| Silica colloidal anhydrous | 0.15-3.0 |
| **Total (First layer)** | **100** |

| **Second Layer** | **(%) amount (w/w)** |
|---|---|
| Ezetimibe | 3.5-10.0 |
| Microcrystalline cellulose | 40.0-50.0 |
| Lactose monohydrate | 27.0-33.0 |
| Croscarmellose sodium | 7.0-13.0 |
| Hydroxypropylmethylcellulose E5 LV | 3.0-8.0 |
| Sodium lauryl sulfate | 1.5-4.0 |

| | |
|---|---|
| Sodium stearyl fumarate | 1.0-3.5 |
| Silica colloidal anhydrous | 0.1-3.0 |
| **Total (Second Layer)** | **100** |

### Process for example 2

### Ezetimibe layer:

a) mixing ezetimibe, sodium lauryl sulfate, hydroxypropylmethylcellulose E5 LV, lactose monohydrate, half of croscarmellose sodium,
b) granulating the powder mixture with pure water
c) drying the granules
d) sieving the dried granules through a sieving mesh
e) adding microcrystalline cellulose, the remaining part of croscarmellose sodium, silica colloidal anhydrous to step (d) mixture and mixing them
f) adding sodium stearyl fumarate to step (e) mixture and mixing them.

### Atorvastatin layer:

a) mixing atorvastatin calcium, microcrystalline cellulose, calcium carbonate, lactose monohydrate, half of croscarmellose sodium,
b) adding hydroxypropylcellulose(LF), polysorbate 80 in aqueous solution to step (a) mixture
c) sieving the wet granulating
d) drying the granules and sieving the mixture
e) adding the remaining part of croscarmellose sodium, silica colloidal anhydrous to step (d) mixture and mixing them
f) adding magnesium stearate to step (e) mixture and mixing them
   - After, pressing these two mixtures for each layer into bilayer tablets,
   - Coating these tablets.

## Claims

1. A pharmaceutical bilayer tablet comprising;
a. first layer comprising atorvastatin calcium
b. second layer comprising ezetimibe
wherein the weight ratio of the second layer to the first layer is between 0.05 and 8.0, further comprising at least one polymer.

2. The pharmaceutical bilayer tablet according to claim 1, wherein the weight ratio of the second layer to the first layer is between 0.1 and 5.0.

3. The pharmaceutical bilayer tablet according to claim 2, wherein the total amount of atorvastatin calcium in the tablet is 0.5% to 10.0% by weight and the total amount of ezetimibe in the tablet is 0.5% to 10.0% by weight

4. The pharmaceutical bilayer tablet according to claim 1, wherein the polymers are selected from the group comprising hydroxypropylcellulose, hydroxypropylmethylcellulose, polysorbate, polyvinylpyrrolidone (PVP), vinyl acetate, cellulose acetate phthalate, polyethylene oxide, polypropylene oxide, copolymers of ethylene oxide, propylene oxide, methacrylic acid/ethyl acrylate copolymers, methacrylic acid/methyl methacrylate copolymers, butyl methacrylate/2-dimethylaminoethyl methacrylate copolymers, polyacrylamides, vinyl acetate polymers, polyvinyl alcohol, hydroxypropylmethylcellulose succinate, hydroxypropylcellulose acetate phthalate, hydroxypropylcellulose acetate succinate, hydroxypropylmethylcellulose acetate phthalate, hydroxypropylmethylcellulose acetate succinate, hydroxypropylmethylcellulose acetate trimellitate, hydroxypropylcellulose butyrate phthalate, methylcellulose acetate phthalate, cellulose acetate trimellitate, cellulose acetate terephthalate or mixtures thereof, preferably the polymers are hydroxypropylcellulose, hydroxypropylmethylcellulose and polysorbate.

5. The pharmaceutical bilayer tablet according to claim 4, wherein the amounts of polymers in the tablet is 0.5% to 5.0% by weight.

6. The pharmaceutical bilayer tablet according to claim 5, wherein each layer comprises at least one polymer.

7. The pharmaceutical bilayer tablet according to claim 6, wherein the weight ratio of polymer in the second layer to polymers in the first layer is 0.1-10.0, preferably the ratio is 0.2-6.0.

8. The pharmaceutical bilayer tablet according to claim 4, wherein the weight ratio of hydroxypropylcellulose to polysorbate is 0.5-10.0, preferably the ratio is 2.1-8.0.

9. The pharmaceutical bilayer tablet according to claim 1, further comprising the fillers which are selected from the group comprising microcrystalline cellulose, lactose monohydrate, starch, sucrose, glucose, dextrose, maltodexrin, natural and synthetic gums, gelatin, pregelatinized starch, polyvinylpyrrolidone, cellulose derivatives or mixtures thereof, preferably the fillers are microcrystalline cellulose and lactose monohydrate.

10. The pharmaceutical bilayer tablet according to claim 9, wherein the amount of the fillers in the tablet is 12.0%-65.0% by weight.

11. The pharmaceutical bilayer tablet according to claim 9, wherein the weight ratio of microcrystalline cellulose in the second layer to microcrystalline cellulose in the first layer is 0.05-10.0, preferably the ratio is 0.1-5.0.

12. The pharmaceutical bilayer tablet according to claim 1, further comprising the stabilizers which are selected from the group comprising calcium carbonate, calcium hydroxide, dibasic calcium phosphate, tribasic calcium phosphate, calcium sulphate, calcium acetate, calcium gluconate, calcium glycerol phosphate, magnesium carbonate, magnesium hydroxide, magnesium sulphate, magnesium acetate, magnesium silicate, magnesium aluminate or mixtures thereof.

13. The pharmaceutical bilayer tablet according to claim 12, wherein the stabilizer is calcium carbonate whose total amount in the tablet is 1.0%- 10.0%, preferably 2.0%-8.0% by weight.

14. The pharmaceutical bilayer tablet according to any preceding claim, wherein the tablet is coated with film-coating, preferably the amount of the film coating is 0.5%-4.0% by weight.

15. The pharmaceutical bilayer tablet according to any preceding claims, wherein the tablet comprising;
a) atorvastatin layer:
a. 1.0-40.0% by weight of atorvastatin calcium,
b. 20.0-50.0% by weight of microcrystalline cellulose,
c. 12.0- 38.0% by weight of calcium carbonate,
d. 12.0-35.0% by weight of lactose monohydrate,
e. 1.0-17.0% by weight of croscarmellose sodium,
f. 0.5-11.0% by weight of hydroxypropylcellulose,
g. 0.01- 5.0 by weight of polysorbate,
h. 0.01-8.0% by weight of magnesium stearate,
i. 0.01-8.0% by weight of silicacolloidal anhydrous,
j. 0.001 -10.0% by weight of pure water in the layer.
b) ezetimibe layer:
a. 1.0-20.0% by weight of ezetimibe,
b. 20.0-60.0% by weight of microcrystalline cellulose,
c. 20.0-42.0% by weight of lactose monohydrate,
d. 3.0-20.0% by weight of croscarmellose sodium,
e. 0.1-12.0% by weight of hydroxypropylmethylcellulose,
f. 0.1-10.0% by weight of sodium lauryl sulfate,
g. 0.1-10.0% by weight of sodium stearyl fumarate,
h. 0.01-8.0% by weight of silica colloidal anhydrous,
i. 0.001 -10.0% by weight of pure water in the layer.

16. A process for preparing the pharmaceutical bilayer tablet according to claim 15, wherein the process comprising the following steps:
Atorvastatin layer:
a) mixing atorvastatin calcium, microcrystalline cellulose, calcium carbonate, lactose monohydrate, half of croscarmellose sodium,
b) mixing polysorbate and pure water
c) adding hydroxypropylcellulose, polysorbate in aqueous solution to step (a) mixture
d) sieving the wet granules
e) drying the granules and sieving the mixture
f) adding the remaining part of croscarmellose sodium, silica colloidal anhydrous to step (e) mixture and mixing them
g) adding magnesium stearate to step (f) mixture and mixing them.
Ezetimibe layer:
a) mixing ezetimibe, sodium lauryl sulfate, hydroxypropylmethylcellulose, lactose monohydrate, half of croscarmellose sodium,
b) granulating the powder mixture with pure water
c) drying the granules
d) sieving the dried granules through a sieving mesh
e) adding microcrystalline cellulose, the remaining part of croscarmellose sodium, silica colloidal anhydrous to step (d) mixture and mixing them
f) adding sodium stearyl fumarate to step (e) mixture and mixing them.
- Then, pressing each layer to form bilayer tablets,
- Coating this bilayer tablets.

17. A process for preparing the pharmaceutical bilayer tablet according to claim 15, wherein the process comprising the following steps:
Atorvastatin layer:
a) mixing atorvastatin calcium, microcrystalline cellulose, calcium carbonate, lactose monohydrate, half of croscarmellose sodium,
b) mixing polysorbate and pure water
c) adding hydroxypropylcellulose, polysorbate in aqueous solution to step (a) mixture
d) sieving the wet granulating
e) drying the granules and sieving the mixture
f) adding the remaining part of croscarmellose sodium, silica colloidal anhydrous to step (e) mixture and mixing them
g) adding magnesium stearate to step (f) mixture and mixing them
Ezetimibe layer:
a) mixing lactose monohydrate and half of croscarmellose sodium,
b) adding hydroxypropylmethylcellulose, sodium lauryl sulfate and ezetimibe in aqueous solution to step (a)
c) drying the granules
d) granulating the powder mixture with pure water
e) drying the granules
f) sieving the dried granules through a sieving mesh
g) adding microcrystalline cellulose, the remaining part of croscarmellose sodium, silica colloidal anhydrous to step (f) mixture and mixing them
h) adding sodium stearyl fumarate to step (g) mixture and mixing them.
- Then, pressing layers to form bilayer tablets,
- Coating this bilayer tablets.
